# EUROPÄISCHE PATENTSCHRIFT

(11) **EP 3 254 989 B1**
(45) Veröffentlichungstag und Bekanntmachung des Hinweises auf die Patenterteilung: **02.10.2019**
(21) Anmeldenummer: 17173922.0
(22) Anmeldetag: 01.06.2017
(51) Int. Cl.: B65D 75/56, A61B 1/00, B65D 77/04, A61B 50/00

(54) **VERPACKUNGSEINRICHTUNG FÜR ZU STERILISIERENDE ODER STERILISIERTE MEDIZINISCHE PRODUKTE MIT INNERER FIXIERUNG**
PACKAGING DEVICE FOR MEDICAL PRODUCTS THAT ARE TO BE STERILIZED OR STERILIZED MEDICAL PRODUCTS WITH INTERNAL FIXING
DISPOSITIF D'EMBALLAGE POUR DES PRODUITS MÉDICAUX STÉRILISÉS OU À STÉRILISER AYANT UNE FIXATION INTERNE

(30) Priorität: 07.06.2016 DE 102016110486
(43) Veröffentlichungstag der Anmeldung: 13.12.2017
(73) Patentinhaber: B. Braun Melsungen AG, 34212 Melsungen (DE)
(72) Erfinder: GEIGER, Ralph, 34587 Felsberg (DE); Dr. van VENROOY-MARKEFKA, Peter, 13589 Berlin (DE)
(74) Vertreter: Winter, Brandl, Fürniss, Hübner, Röss, Kaiser, Polte - Partnerschaft mbB

(56) Entgegenhaltungen:
- WO-A1-2011/070329
- DE-A1-102006 027 304
- DE-T2- 69 938 259

## Beschreibung

Die Erfindung betrifft eine Verpackungseinrichtung mit einem steril verschließbaren Aufnahmeraum für zu sterilisierende oder sterilisierte medizinische Produkte und einem Träger zum Aufhängen und/oder Führen und/oder Hantieren der Verpackungseinrichtung. Außerdem betrifft die Erfindung eine Sekundärverpackung zur Aufnahme einer Vielzahl von derartigen Verpackungseinrichtungen.

Medizinische Produkte wie beispielsweise Schlauchsysteme, Katheter oder intravenöse Sets werden grundsätzlich in Sterilverpackungen verpackt, darin sterilisiert und anschließend ausgeliefert. Eine Sterilverpackung muss dabei zum einen eine Sterilisation ermöglichen und zum anderen die Sterilität bei geeigneter Lagerung bis zur Anwendung der darin aufgenommenen medizinischen Produkte gewährleisten. Es muss generell eine Rekontamination des sterilisierten Medizinprodukts nach seiner Aufbereitung bis zu seiner Anwendung ausgeschlossen sein. Medizinische Einmal-/ bzw. Einwegprodukte, wie beispielsweise Blutschläuche, oder chirurgische Instrumente werden dabei in aus dem Stand der Technik bekannter Weise in Beuteln oder Blisterverpackungen verpackt und anschließend sterilisiert. Die Sterilisation kann in einer Einzelverpackung, in einem Spenderkarton oder gestapelt auf einer Palette erfolgen. Bei den aus dem Stand der Technik bekannten Verpackungen sind die Produkte dabei grundsätzlich lose, das heißt nicht fixiert innerhalb der (Primär-) Verpackung/ eines Beutels/ einer Blisterverpackung angeordnet.

Die lose Anordnung der zu sterilisierenden oder sterilisierten medizinischen Produkte in der Verpackung hat jedoch den Nachteil, dass bei einer Handhabung/ einem Handling und einem Transport die verpackten medizinischen Produkte insbesondere durch Verformungen, Knicke oder Beschädigungen der Verpackung, beschädigt oder beeinträchtigt werden. Außerdem kann es durch die lose Anordnung der medizinischen Produkte in einem Beutel bei einer Entnahme von mehreren lose gepackten Produkten wie zum Beispiel Blutschläuchen zu Verunreinigungen kommen, da zumindest Abschnitte der Produkte beim Entnehmen Kontakt zum Boden haben können.

Ferner kommt es durch die lose Anordnung innerhalb der Verpackung beispielsweise während der Elektronenstrahl-Sterilisation (sogenannte E-Beam-Technologie) zu einer ungleichen Dosisverteilung bedingt durch eine hohe Streustrahlung, welche zu einer inhomogenen Belastung der medizinischen Produkte führt. Die Elektronenstrahl-Sterilisation beruht auf der Durchdringung der zu sterilisierenden medizinischen Produkte inklusive der Verpackung durch hochenergetische beschleunigte Elektronen, welche eine ionisierende Wirkung haben und somit keimabtötend sind. Wird nun bei der Elektronenstrahl-Sterilisation aufgrund der Streustrahlung eine geforderte Mindestbestrahlung unterschritten, führt dies zu einer Unsterilität. Eine Überbestrahlung in Form einer zu hohen energetischen Belastung der zu sterilisierenden medizinischen Produkte führt auf der anderen Seite zu Materialveränderungen wie beispielsweise Verfärbungen, Verschlechterung der mechanischen Eigenschaften oder Alterung. Es ist somit durch die lose Anordnung der zu sterilisierenden medizinischen Produkte im Falle der Strahlensterilisation eine minimierte Bestrahlung des einzelnen Medizinprodukts nicht möglich. Aufgrund der entstehenden hohen Streuung muss eine Leistung der Strahlenquelle hoch sein, um eine Mindestbestrahlung und somit eine Sterilität zu garantieren. Eine optionale Gassterilisiation erzeugt auf der anderen Seite hohe Restgaswerte und benötigt zusätzlich eine gasdurchlässige Verpackung.

Weitere Nachteile des Stands der Technik sind, dass die Verpackung nach der Sterilisation in einem zusätzlichen Fertigungsschritt noch mit einem Etikett zur Beschriftung versehen werden muss und eine automatisierte Herstellung bzw. Verpackung der medizinischen Produkte nur bedingt möglich ist.

Eine Verpackungseinrichtung gemäß dem Oberbegriff des Anspruchs 1 ist in der DE 699 38 259 T2 gezeigt. Weiterer relevanter Stand der Technik findet sich in der WO 2011/070329 A1 und der DE 10 2006 027304 A1.

Es ist also die Aufgabe der Erfindung, die Nachteile aus dem Stand der Technik zu vermeiden oder wenigstens zu mildern. Insbesondere soll eine Verpackungseinrichtung, welche eine lose Anordnung und ein damit einhergehendes Verrutschen von Medizinprodukten innerhalb dieser vermeidet, bereitgestellt werden. Die Verpackungseinrichtung soll insbesondere eine Prozessverarbeitung während der Sterilisation optimieren und eine Haltbarkeit, Stabilität und eine Kennzeichnung der Verpackung verbessern. Außerdem soll ein Transport einer Vielzahl von derartigen Verpackungseinrichtungen optimiert werden.

Diese Aufgaben werden insbesondere durch eine Verpackungseinrichtung gemäß den Merkmalen des Anspruchs 1 aber auch durch eine Sekundärverpackung gemäß den Merkmalen des Anspruchs 9 gelöst. Vorteilhafte Weiterbildungen sind Gegenstand der Unteransprüche.

Die Erfindung betrifft zunächst eine Verpackungseinrichtung mit einem steril verschließbaren Aufnahmeraum für zu sterilisierende oder sterilisierte medizinische Produkte und einem Träger zum Aufhängen und/oder Führen und/oder Hantieren der Verpackungseinrichtung, wobei der Träger in das Innere des Aufnahmeraums hineinreicht und zumindest eine passgenaue Aufnahme für einen Abschnitt eines zu sterilisierenden oder sterilisierten medizinischen Produkts aufweist.

Dabei ist unter dem Aufnahmeraum ein steril abgeschlossenes Volumen zu verstehen, in welchem sich die sterilisierten oder zu sterilisierenden medizinischen Produkte befinden. Der Träger ist erfindungsgemäß außerhalb und innerhalb des Aufnahmeraums vorgesehen und dient außerhalb des Aufnahmeraums einem Aufhängen, Führen, Hantieren bzw. einem geeigneten, präzisen Orientieren der Verpackungseinrichtung/ der Verpackung und innerhalb des Aufnahmeraums einer Fixierung der zu sterilisierenden oder sterilisierten medizinischen Produkte und einer Vermeidung von Druckstellen an der Verpackung.

Durch eine derartige Verpackungseinrichtung wird erreicht, dass die zu sterilisierenden oder sterilisierten medizinischen Produkte nicht mehr lose in der Verpackung bzw. dem Aufnahmeraum vorliegen, sondern an einem Träger innerhalb des Aufnahmeraums bzw. innerhalb der Verpackung aufgenommen/ fixiert/ befestigt sind. Dadurch können die medizinischen Produkte in einer vorbestimmten Lage positioniert bzw. angeordnet werden, so dass eine definierte Position innerhalb der Verpackungseinrichtung erreicht werden kann. Außerdem wird eine wiederholbare Anordnung bzw. Positionierung der medizinischen Produkte ermöglicht und ein Verrutschen dieser minimiert bzw. ausgeschlossen.

Es werden somit erfindungsgemäß die oben beschriebenen Nachteile einer losen Anordnung der medizinischen Produkte in der Verpackung vermieden. Insbesondere führt eine derartige Verpackungseinrichtung zu einem geeigneteren Transport, einer verbesserten Handhabung, Orientierung, Etikettierung und Automatisierung. Ferner ist die erfindungsgemäße Verpackungseinrichtung besser an die Anforderungen an eine Verpackungseinrichtung in der Strahlensterilisation, insbesondere der Elektronenstrahl-Sterilisation angepasst. Durch die Fixierung an dem Träger wird zudem einer möglichen Kontaminierung der medizinischen Produkte bei deren Entnahme entgegengewirkt.

Es ist dabei zweckmäßig, wenn der Träger/ das Trägersystem einen Außenführungsabschnitt, welcher insbesondere zum Aufhängen und/oder Führen und/oder Hantieren der Verpackungseinrichtung ausgebildet ist, und einen Innenaufnahmeabschnitt, welcher insbesondere zur passgenauen Aufnahme des zu sterilisierenden oder sterilisierten medizinischen Produkts ausgebildet ist, aufweist, wobei der Außenführungsabschnitt außerhalb des Aufnahmeraums und der Innenaufnahmeabschnitt innerhalb des Aufnahmeraums angeordnet ist und der Innenaufnahmeabschnitt und der Außenführungsabschnitt einstückig, vorzugsweise einmaterilig und integral, insbesondere als ein im Spritzgussverfahren hergestelltes Kunststoffteil ausgebildet sind.

Es werden somit mit dem erfindungsgemäßen Träger Funktionen bzw. technische Effekte innerhalb und außerhalb des Aufnahmeraums realisiert. Der Träger, welcher aus dem Außenführungsabschnitt und dem Innenaufnahmeabschnitt besteht, ist ein einstückiges, vorzugsweise einmaterialiges oder integrales Bauteil und somit in einfacher Weise sowie kostengünstig im Spritzgussverfahren herstellbar. Der Träger besteht aus einem ausreichend strahlenstabilen sowie steifen und festen Kunststoff und kann Markierungen, insbesondere Lasermarkierungen, aufweisen, welche Anweisungen an einen Anwender enthalten können.

Ferner ist es vorteilhaft, wenn der Aufnahmeraum durch eine Folienverpackung, insbesondere nach Art einer Folientasche mit einer Lasche zum Verschließen der Folientasche, ausgebildet ist, wobei an einer Schnittstelle, an der der Träger in das Innere der Folienverpackung übergeht, eine stoffschlüssige Verbindung, insbesondere durch Verschweißen oder Verkleben, zwischen dem Träger und der Folienverpackung ausgebildet ist. Es wird somit durch die stoffschlüssige Verbindung an der Schnittstelle zwischen dem Träger und der Folienverpackung ein steriles Verschließen des Aufnahmeraums erreicht. Dies hat den Vorteil, dass eine Re-Kontaminierung nach der Sterilisation quasi ausgeschlossen ist.

In anderen Worten besteht die Verpackungseinrichtung bzw. Primärverpackung somit aus einem Träger/ einem Trägersystem und einer Folienverpackung/ einer Folientasche, wobei die insbesondere biegeschlaffe Folienverpackung/ Folientasche in ihrem Inneren den Aufnahmeraum der vorliegenden Erfindung ausbildet. Die Folienverpackung/ Folientasche besteht erfindungsgemäß aus einem ausreichend strahlenstabilen und/oder druckstabilen Kunststoff. Sie ist frei gestaltbar und kann beispielsweise vorbedruckt sein und/oder eine UDI (Unique Device Identification) und/oder eine IFU (Instruction for Use) aufweisen. Die Folienverpackung verschließt die an dem Träger vorgesehene Aufnahme. Die Folienverpackung kann auch auf den Träger geklebt oder laminiert sein und/oder eng anliegend an den Träger ausgebildet sein, so dass die erfindungsgemäße Fixierung der zu sterilisierenden oder sterilisierten medizinischen Produkte zusätzlich auch über die Folienverpackung erfolgen kann.

Ein vorteilhaftes Ausführungsbeispiel ist dadurch gekennzeichnet, dass der Träger eine Evakuierungseinrichtung, insbesondere nach Art eines Vakuumkanals, aufweist, über welche zumindest ein Teilvakuum, vorzugsweise ein Vakuum, in dem Aufnahmeraum erzeugbar ist. Dadurch wird erreicht, dass Luft, welche in dem Aufnahmeraum/ innerhalb der Folienverpackung/ Folientasche eingeschlossen ist, aus dem Aufnahmeraum herausgezogen bzw. abgesaugt wird. Dies hat zunächst den Vorteil, dass die in dem Aufnahmeraum angeordneten medizinischen Produkte durch das Teilvakuum oder Vakuum zusätzlich befestigt bzw. fixiert werden. Außerdem wird durch das Teilvakuum oder Vakuum das Verpackungsvolumen in großem Maße reduziert.

Aufgrund von eingeschlossener Luft kommt es bei der Strahlensterilisation häufig zu einer Ozonbildung innerhalb der Verpackung, welche die verpackten Produkte schädigen, bzw. eine Ozonbelastung des Benutzers zur Folge haben kann, so dass durch ein Anlegen eines (Teil-) Vakuums dieser Nachteil beseitigt werden kann. Das Vorsehen einer Evakuierungseinrichtung liefert somit Vorteile bei der Fixierung der medizinischen Produkte, hinsichtlich des Verpackungsvolumens sowie bei der Strahlensterilisation. Es versteht sich von selbst, dass die Evakuierungseinrichtung eine Verschlussmöglichkeit in Form einer Verschlusseinrichtung aufweisen muss, damit der Aufnahmeraum weiterhin steril verschließbar ist.

Außerdem ist es von Vorteil, wenn der Außenführungsabschnitt als ein Führungshaken, insbesondere nach Art eines Kleiderbügelhakens oder T-förmig mit zwei seitlichen Ausnehmungen, zur geeigneten Orientierung und/oder Handhabung und/oder Führung der Verpackungseinrichtung ausgebildet ist. Der Träger kann somit einen T-förmigen Führungshaken mit zwei seitlichen Ausnehmungen aufweisen, über welche der Führungshaken in beispielsweise eine oder zwei Führungsschienen einklipsbar bzw. einrastbar ist. Alternativ kann der Träger einen kleiderbügelartigen Führungshaken aufweisen, welcher beispielsweise an einer Führungsschiene aufhängbar ist. Die Verpackungseinrichtung ist somit beispielsweise an einer Führungsschiene aufhängbar bzw. anbringbar. Die erfindungsgemäße Evakuierungseinrichtung/ der Vakuumkanal kann dabei in dem Führungshaken vorgesehen sein.

Der Führungshaken ermöglicht eine geeignete Führung sowohl während der Produktion bzw. Herstellung als auch während der Sterilisation, eine geeignete Orientierung sowie eine automatisierte Handhabung der Verpackungseinrichtung. Es werden somit mit Hilfe des Trägers bzw. des Führungshakens ein hängender oder geführter Transport und eine präzise Zuführung in eine Sterilisationseinrichtung erreicht. Der Träger bzw. der Führungshaken ermöglicht somit eine Automatisierung des Sterilisationsprozesses und einen verbesserten Materialfluss, insbesondere durch ein mögliches Vorsehen eines Puffers und eine damit einhergehende Vermeidung von Stoppzeiten. Der Führungshaken befindet sich außerhalb des Aufnahmeraums und stellt somit einen unsterilen Abschnitt des Trägers bzw. der Trägerstruktur dar.

Der Führungshaken kann Markierungen in Form von Laserkennzeichnungen, Farbcodierungen, etc. oder daran angebrachte Etikette aufweisen, welche Anweisungen für einen Anwender beinhalten. Diese Anweisungen können derart sein, dass ein Anwender unter Beachtung der angebrachten Markierungen, Codierungen, Kennzeichnungen oder Etikette im Grunde keine Fehler mehr machen kann, so dass eine Fehlervermeidungseinrichtung/ ein Fehlervermeidungssystem in dem Führungshaken vorgesehen sein kann.

Die Erfindung ist dabei dadurch gekennzeichnet, dass die zumindest eine Aufnahme für einen Abschnitt eines zu sterilisierenden oder sterilisierten medizinischen Produkts nach Art eines Konnektors ausgebildet ist, welcher die zu sterilisierenden oder sterilisierten medizinischen Produkte in dem Aufnahmeraum aufnimmt, orientiert und fixiert, wobei der Konnektor eine, insbesondere u-förmige, Ausnehmung zum formschlüssigen Befestigen, insbesondere Einklipsen oder Einrasten, der zu sterilisierenden oder sterilisierten medizinischen Produkte aufweist. Der Träger weist somit innerhalb des Aufnahmeraums bzw. der Folienverpackung/ Folientasche Konnektorabschnitte bzw. Konnektor-Clips auf, in welche Abschnitte der zu sterilisierenden oder sterilisierten medizinischen Produkte einklipsbar bzw. einrastbar sind. Auch ein automatisiertes Einklipsen bzw. Einrasten ist erfindungsgemäß denkbar. Die Konnektorabschnitte können beispielsweise als Luer-Lock-Konnektoren, Hansen-Konnektoren oder Small-Bore-Konnektoren ausgebildet sein.

Die medizinischen Produkte sind somit formschlüssig durch Einklipsen oder Einrasten an dem Träger befestigt, so dass eine einfache Befestigung/ ein einfaches Anbringen sowie eine einfache Entnahme der medizinischen Produkte ermöglicht werden. Die Konnektorabschnitte ermöglichen die Fixierung der medizinischen Produkte an dem Träger und eine präzise Orientierung dieser während der Sterilisation. Die Konnektorabschnitte können derart ausgebildet sein, dass Abschnitte von Medizinprodukten passgenau in ihnen aufgenommen werden können. Beispielsweise können die Konnektorabschnitte als passgenaue Ausnehmungen ausgebildet sein. Auch können sich die Aufnahmen bzw. Konnektorabschnitte an einem Träger in ihrer Form und Gestalt unterscheiden, so dass jede Aufnahme bzw. jeder Konnektor passgenau für den darin aufzunehmenden Abschnitt des Medizinprodukts ausgebildet sein kann. Es wird somit eine für den Anwendungsfall geeignete Aufnahme der Medizinprodukte an dem Träger bereitgestellt. Durch die Fixierung wird zudem ein Bodenkontakt bei der Entnahme verhindert.

Es ist zweckmäßig, wenn die zumindest eine Aufnahme für einen Abschnitt eines zu sterilisierenden oder sterilisierten medizinischen Produkts eine Kennzeichnung aufweist und/oder die Aufnahme in ihrer Form und/oder Gestalt an den Abschnitt des in ihr aufgenommenen zu sterilisierenden oder sterilisierten medizinischen Produkts angepasst ist. Somit wird erreicht, dass ein Anwender durch eine Kennzeichnung wie etwa eine Farbmarkierung, oder durch die Form und/oder Gestalt der Aufnahme weiß, welches medizinische Produkt bzw. welcher Abschnitt des medizinischen Produkts in eben dieser Aufnahme angebracht bzw. fixiert werden muss.

Beispielsweise können in einem Fall, in dem zwei Schläuche, nämlich eine Blutleitung und eine Spülleitung in der Verpackung vorgesehen sind, rote Markierungen für die Blutleitung und blaue Markierungen für die Spülleitung angebracht sein, so dass ein Anwender bei der Entnahme der Schläuche die Blutleitungsanschlüsse im Grunde nicht mit den Spülleitungsanschlüssen verwechseln kann. Als Markierungen sind grundsätzlich Farben, Formen, Worte, etc. denkbar. Dies hat den Vorteil, dass Fehler eines Anwenders bei der Fixierung, der Entnahme und der Anwendung verhindert werden können. Es ist somit eine Fehlervermeidungseinrichtung auch innerhalb des Aufnahmeraums an dem Träger vorgesehen.

In vorteilhafter Weise weist der Träger in dem Inneren des Aufnahmeraums ferner zumindest zwei, vorzugsweise drei oder vier, den Aufnahmeraum aufspannende Stapelabschnitte/ Stapel-Clips zur geeigneten Stapelung und/oder Führung der Verpackungseinrichtung auf. Dies hat den Effekt, dass innerhalb des Aufnahmeraums Abschnitte vorgesehen sind, welche eine vereinfachte Stapelung bzw. Stapelbarkeit der Verpackungseinrichtung ermöglichen. Dies hat zum einen den Vorteil einer verringerten mechanischen Belastung und somit einer verbesserten Aufbewahrung einer Vielzahl von erfindungsgemäßen Verpackungseinrichtungen in einer Sekundärverpackung wie einem Karton oder Aufnahmebehälter. Zum anderen werden beispielsweise während der Sterilisation eine geeignete Führung und Orientierung der Verpackungseinrichtung ermöglicht. Ferner werden durch die Stapelabschnitte Druckstellen an der Verpackung verhindert.

Es ist außerdem vorteilhaft, wenn der Träger in dem Inneren des Aufnahmeraums u-förmig oder hufeisenförmig zum seitlichen Schutz der in dem Aufnahmeraum aufgenommenen zu sterilisierenden oder sterilisierten medizinischen Produkte ausgebildet ist. Durch eine derartige Ausbildung des Trägers wird die Stabilität der Verpackungseinrichtung in großem Maße erhöht, die medizinischen Produkte werden geschützt und somit eine Haltbarkeit der Verpackungseinrichtung verbessert. Außerdem können Druckstellen durch eine derartige Ausbildung des Trägers verhindert werden.

Mit anderen Worten betrifft die Erfindung zunächst eine Verpackung mit einem Trägersystem zur inneren Fixierung von verpackten Produkten, insbesondere Medizinprodukten. Eine lose Anordnung von Medizinprodukten in einer Sterilverpackung ist in vielerlei Hinsicht nachteilig. Die Nachteile des Stands der Technik werden durch die Fixierung der Medizinprodukte innerhalb der erfindungsgemäßen Verpackung behoben. Insbesondere wird durch ein Trägersystem eine direkte Sterilisation einer Einzelverpackung durch Strahlensterilisation, insbesondere durch Elektronenstrahl-Sterilisation (sogenannte E-Beam-Technologie) ermöglicht. Die Verpackungsoptimierung der vorliegenden Erfindung ermöglicht eine verkürzte Bestrahlungszeit, eine geringere Streustrahlung sowie eine geringere energetische Belastung. Die Verpackungseinrichtung der vorliegenden Erfindung ist somit insbesondere an die Anforderungen der E-Beam-Technologie angepasst, jedoch bietet die Fixierung wie erwähnt auch Vorteile bei anders sterilisierten medizinischen Produkten.

Die Erfindung betrifft ferner eine Sekundärverpackung mit einer Vielzahl von erfindungsgemäßen Verpackungseinrichtungen, wobei die Sekundärverpackung nach Art eines Aufnahmebehälters ausgebildet ist und eine Führungsschiene aufweist, an welcher die Träger der Verpackungseinrichtungen anbringbar sind. Es wird somit eine geeignete Anordnung, eine feste Orientierung, eine einfache Entnahme, eine geringe mechanische Belastung, insbesondere während des Transports und eine automatisierte Verpackung der erfindungsgemäßen Verpackungseinrichtungen in einer Sekundärverpackung erreicht. Dies hat den Vorteil, dass die Anzahl der verbleibenden Verpackungseinrichtungen mit den darin aufgenommenen medizinischen Produkten zu jeder Zeit direkt sichtbar ist. Dies ist bei losen Verpackungen, welche ungeordnet in einer Sekundärverpackung liegen, nicht der Fall.

Dabei ist es zweckmäßig, wenn eine Höhe und Breite der Sekundärverpackung bzw. des Aufnahmebehälters den äußeren Abmessungen der erfindungsgemäßen Verpackungseinrichtung entsprechend ausgebildet ist. Dadurch kann nämlich das Volumen der Sekundärverpackung in großem Maße reduziert werden. Erfindungsgemäß wird somit auch ein Transport der in der Sekundärverpackung aufgenommenen Verpackungseinrichtungen erheblich verbessert.

Die Erfindung wird nachfolgend mit Hilfe von Zeichnungen weiter erläutert. Es zeigen:
- Fig. 1: eine Vorderansicht eines ersten Beispiels einer Verpackungseinrichtung;
- Fig. 2: eine Vorderansicht eines Trägers der Verpackungseinrichtung mit daran aufgenommenen medizinischen Schläuchen;
- Fig. 3: eine Vorderansicht einer einen Aufnahmeraum ausbildenden Folientasche;
- Fig. 4: eine Vorderansicht eines zweiten Beispiels einer Verpackungseinrichtung;
- Fig. 5: eine Vorderansicht des Trägers mit daran aufgenommenen medizinischen Schläuchen;
- Fig. 6: eine Vorderansicht einer erfindungsgemässen konstruktiven Ausbildung des Trägers des ersten Beispiels;
- Fig. 7: eine Draufsicht des Trägers der Fig. 6 mit Folientasche;
- Fig. 8: eine Vorderansicht einer weiteren möglichen konstruktiven Ausbildung des Trägers gemäss der Erfindung;
- Fig. 9: eine Draufsicht des Trägers der Fig. 8 mit Folientasche;
- Fig. 10: eine perspektivische Ansicht eines erfindungsgemäßen Konnektorabschnitts
- Fig. 11: eine perspektivische Ansicht einer ersten Ausführungsform eines erfindungsgemäßen Stapelabschnitts;
- Fig. 12: eine perspektivische Ansicht einer zweiten Ausführungsform eines erfindungsgemäßen Stapelabschnitts;
- Fig. 13: eine perspektivische Ansicht einer erfindungsgemäßen Sekundärverpackung mit darin aufgenommenen erfindungsgemäßen Verpackungseinrichtungen; und
- Fig. 14: eine Draufsicht auf eine erfindungsgemäße Sekundärverpackung mit darin aufgenommenen erfindungsgemäßen Verpackungseinrichtungen.

Die Figuren sind lediglich schematischer Natur und dienen ausschließlich dem Verständnis der Erfindung. Gleiche Elemente sind mit denselben Bezugszeichen versehen. Die Merkmale der einzelnen Ausführungsbeispiele und Ausführungsformen können untereinander ausgetauscht werden, wobei der Schutzbereich durch die Merkmalskombinationen der Ansprüche festgelegt wird.

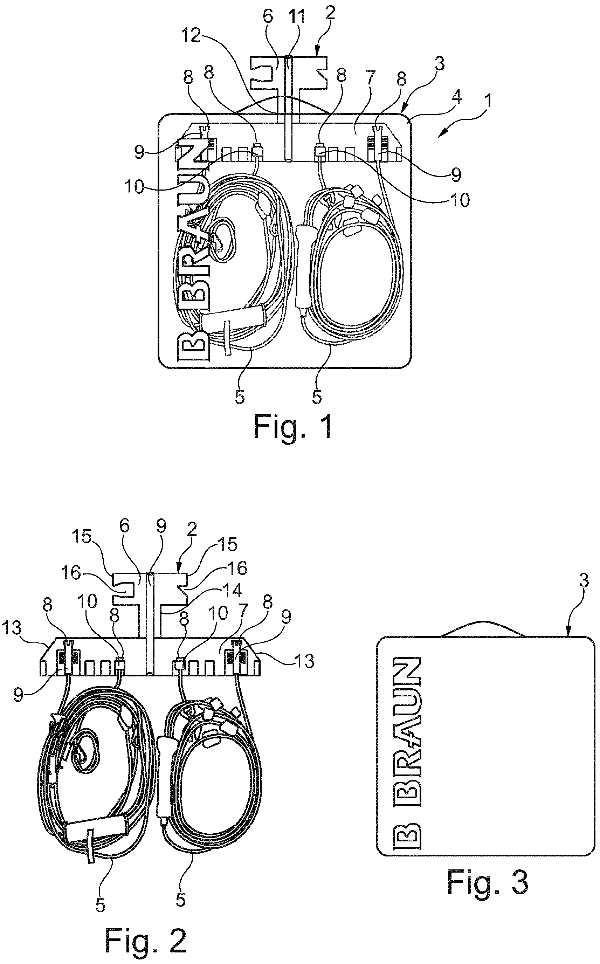

In Fig. 1 ist eine Vorderansicht eines ersten Beispiels einer Sterilverpackung 1 gezeigt. Die Sterilverpackung 1 weist einen Träger 2 und eine Folientasche 3 auf. Innerhalb der Folientasche 3, das heißt in einem Verpackungsinnenraum 4, befinden sich zwei aufgewickelte medizinische Schläuche 5. Der Träger 2 ist außerhalb der Folientasche 3 als ein T-förmiger Führungshaken 6 ausgebildet und reicht in den Verpackungsinnenraum 4 hinein. In dem Verpackungsinnenraum 4 weist der Träger 2 einen Aufnahmeabschnitt 7 mit vier Aufnahmen 8 auf, wobei in diesen ein Anfangsanschlussstecker 9 (mit einem Luer-Lock) und ein Endanschlussstecker 10 (mit einem Luer-Lock) des ersten medizinischen Schlauchs 5 (Blutschlauchsystem für Hämodialyse) sowie ein Anfangsanschlussstecker 9 und ein Endanschlussstecker 10 (jeweils ebenso mit Luer-Lock) des zweiten medizinischen Schlauchs 5 eingeklipst sind. Die Aufnahmen 8 unterscheiden sich und sind passgenau für den Anfangsanschlussstecker 9 bzw. den Endanschlussstecker 10 ausgebildet. Der Aufnahmeabschnitt 7 ist an einem oberen Abschnitt der Folientasche 3 angeordnet. Durch das Aufwickeln der medizinischen Schläuche 5 und das Einklipsen der Anfangsanschlussstecker 9 und Endanschlussstecker 10 der Schläuche 5 in die Aufnahmen 8 ist eine sichere Fixierung der beiden Schläuche 5 gewährleistet. Der Träger 2 weist ferner einen Vakuumkanal 11 auf, welcher von außen in den Verpackungsinnenraum 4 hineinreicht. Die Folientasche 3 ist durch eine nicht dargestellte Lasche verschlossen. An einer Schnittstelle 12 sind der Träger 2 und die Folientasche 3 stoffschlüssig miteinander verbunden.

In Fig. 2 ist eine Vorderansicht des Trägers 2 der Sterilverpackung 1 mit daran aufgenommenen medizinischen Schläuchen 5 gezeigt. Die Schläuche 5 sind aufgewickelt und die Anfangsanschlussstecker 9 sowie Endanschlussstecker 10 der Schläuche 5 sind in die vier Aufnahmen 8 eingeklipst. Das Einklipsen kann beliebig von oben, vorne, unten oder seitlich erfolgen. Der Aufnahmeabschnitt 7 des Trägers 2 in dem Verpackungsinnenraum 4 ist näherungsweise rechteckförmig in der Vorderansicht ausgebildet, weist jedoch an den beiden, in Fig. 2 oberen, Ecken, welche mit Ecken der Folientasche 3 zusammenfallen, jeweils Fasen 13 auf. Durch die Fasen 13 wird eine Beschädigung der Folientasche 10 aufgrund von spitzen Ecken verhindert. Die Länge des Aufnahmeabschnitts 7 entspricht näherungsweise der Länge der oberen Kante der Folientasche 3 in der Vorderansicht. Die Breite des Aufnahmeabschnitts 7 ist im Vergleich zur Länge gering. Der T-förmige Führungshaken 6 erstreckt sich von einer Mitte des Aufnahmeabschnitts 7 nach außen bzw. oben hin weg. Der Führungshaken 6 besteht aus einem Halsabschnitt 14, welcher senkrecht zu dem Aufnahmeabschnitt 7 angeordnet ist und zwei Führungsschienenaufnahmeabschnitte 15, welche durch seitliche Ausnehmungen 16 in dem Führungshaken 6 ausgebildet sind. Der Halsabschnitt 14 sowie die Führungsschienenaufnahmeabschnitte 15 bilden zusammen die T-Form des Führungshakens 6 aus. Der Führungshaken 6 der Fig. 2 kann entweder lediglich einseitig an eine nicht dargestellte Führungsschiene eingeklipst werden, oder zweiseitig geführt sein, oder an einer Seite eingeklipst und an der anderen Seite geführt sein. Der Vakuumkanal 9 erstreckt sich mittig durch den Führungshaken 6 und den Aufnahmeabschnitt 7 und ist rohrartig in dem Träger 2 ausgebildet.

In Fig. 3 ist eine Vorderansicht der den Verpackungsinnenraum 4 ausbildenden, - biegeschlaffen Folientasche 3 dargestellt. Die Folientasche 3 der Fig. 3 ist bedruckt.

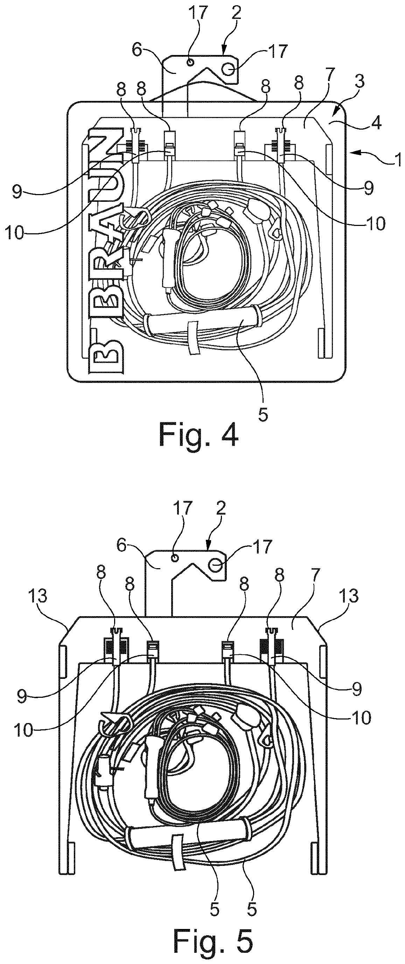

In Fig. 4 ist eine Vorderansicht eines zweiten Ausführungsbeispiels der erfindungsgemäßen Sterilverpackung 1 gezeigt. Die Sterilverpackung 1 unterscheidet sich von der des ersten Ausführungsbeispiels lediglich in der Ausbildung des Trägers 2, so dass im Folgenden nur auf die Unterschiede eingegangen wird. Der Träger 2 ist ohne Folientasche 3 in Fig. 5 gezeigt. Der Führungshaken 6 ist kleiderbügelhakenartig ausgebildet und weist zwei Kreisausnehmungen 17 auf. Die Kreisausnehmungen 17 sind kreisförmig, jedoch sollen sie nicht auf eine Kreisform beschränkt sein und können auch dreieckig, viereckig, trapezförmig, oval, etc. ausgebildet sein. Der Träger 2 ist somit analog zu einem Kleiderbügel über den Führungshaken 6 an einer nicht dargestellten Führungsschiene anbringbar bzw. aufhängbar. Zusätzlich oder alternativ kann der Träger 2 auch über zumindest eine der Kreisausnehmungen 17, welche in dem Führungshaken 6 vorgesehen sind, geführt sein. Der Führungshaken 6 erstreckt sich von dem Aufnahmeabschnitt 7 senkrecht nach außen und ist mittig angeordnet. Der Aufnahmeabschnitt 7 ist u-förmig ausgebildet, so dass sich der Aufnahmeabschnitt 7 u-förmig entlang der oberen Kante sowie entlang der seitlichen Kanten der Folientasche 3 erstreckt. Dadurch werden die in dem Verpackungsinnenraum 4 aufgenommenen medizinischen Schläuche 5 seitlich geschützt.

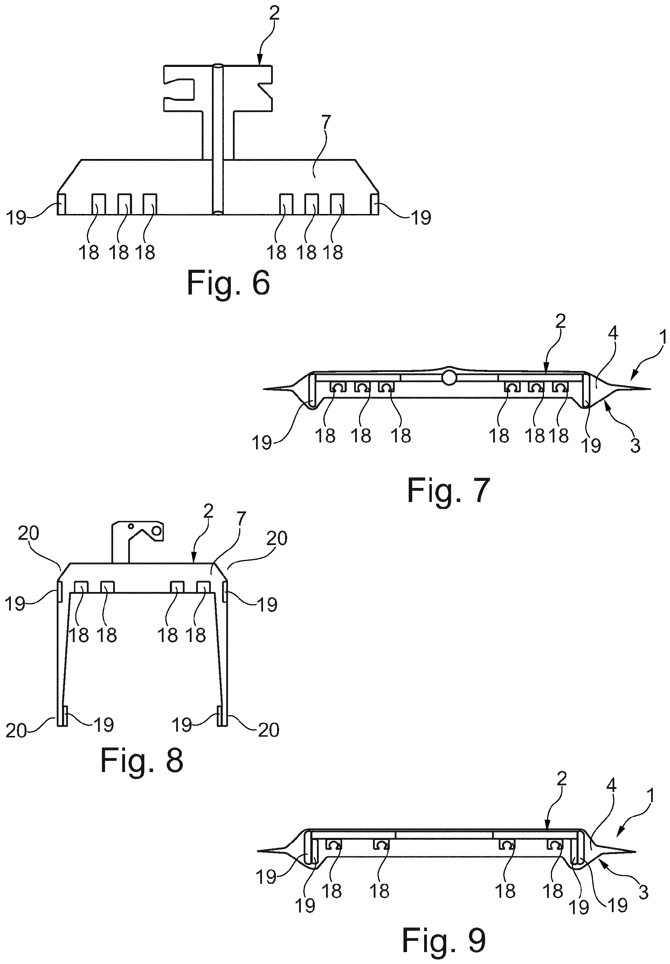

In Fig. 6 ist eine Vorderansicht einer erfindungsgemässen konstruktiven Ausbildung des Trägers 2 des ersten Ausführungsbeispiels gezeigt. Der Träger 2 weist hier an dem Aufnahmeabschnitt 7 sechs Konnektor-Clips 18, welche die erfindungsgemäßen Aufnahmen 8 ausbilden, sowie zwei Stapel-Clips 19 auf. Die Stapel-Clips 19 dienen dabei einer Stapelung und einem Führen der gesamten Sterilverpackung 1 und sind jeweils außenliegend an dem Aufnahmeabschnitt 7 angeordnet. Die Länge des Aufnahmeabschnitts 7 entspricht in anderen Worten etwa dem Abstand der Stapel-Clips 19. In Fig. 7 ist eine Draufsicht des Trägers 2 der Fig. 6 mit Folientasche 3 gezeigt. Es ist erkennbar, dass die Konnektor-Clips 18 u-förmig/ hufeisenförmig in der Draufsicht ausgebildet sind. Die Stapel-Clips 19 spannen den Verpackungsinnenraum 4 auf, das heißt die Stapel-Clips 19 bilden die maximale Erstreckung des Aufnahmeabschnitts 7 in Tiefenrichtung und die Folientasche 3 ist eng an die Stapel-Clips 19 anliegend angeordnet. Das Anlegen der Folientasche 3 an die Stapel-Clips 19 kann durch Ziehen eines Teilvakuums oder Vakuums über den Vakuumkanal 11 noch verstärkt werden. Abgesehen von den Stapel-Clips 19 ist die Tiefe bzw. Dicke des Aufnahmeabschnitts 7 wie auch des gesamten Trägers 2 gering, um die Masse der erfindungsgemäßen Sterilverpackung nicht unnötig zu erhöhen.

In Fig. 8 ist eine Vorderansicht einer weiteren erfindungsgemässen, konstruktiven Ausbildung des Trägers 2 für des zweite Ausführungsbeispiel der Verpackungseinrichtung gezeigt. Der Aufnahmeabschnitt 7 des Trägers 2 weist hier vier Konnektor-Clips 18 sowie vier Stapel-Clips 19 auf. Die Stapel-Clips 19 sind an Eck- bzw. Endpunkten 20 des u-förmigen Aufnahmeabschnitts 7 vorgesehen, so dass die Stapel-Clips 19 über die Sterilverpackung 1 gleichmäßig verteilt und jeweils an Ecken der Sterilverpackung 1 angeordnet sind. Durch das Vorsehen von vier Stapel-Clips 19 in dieser Ausführungsform kann im Vergleich zur ersten Ausführungsform eine Stapelung und Führung der gesamten Sterilverpackung 1 in großem Maße verbessert werden, da nun vier Stapelpunkte bzw. Stapelflächen vorgesehen sind und nicht nur zwei, so dass eine statische Überbestimmung vorliegt. In Fig. 9 ist eine Draufsicht des Trägers 2 der Fig. 8 mit Folientasche 3 gezeigt. Auch in dieser Ausführungsform spannen die Stapel-Clips 19 den Verpackungsinnenraum 4 auf. Die Folientasche 3 ist somit auch hier eng anliegend an die Stapel-Clips 19 angeordnet.

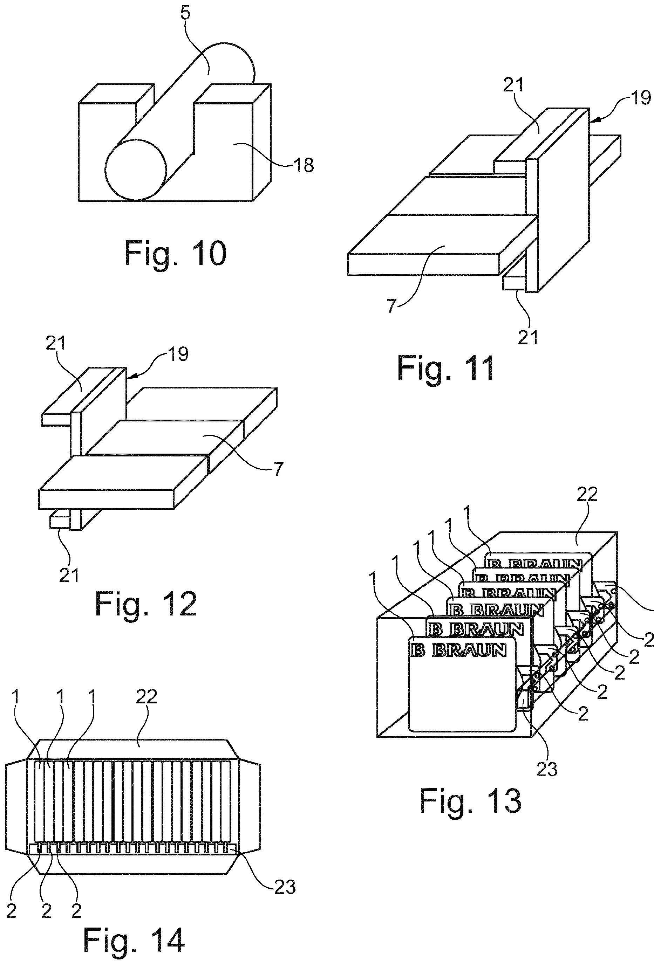

In Fig. 10 ist eine perspektivische Ansicht eines erfindungsgemäßen Konnektor-Clips 18 dargestellt. Der Konnektor-Clip 18 ist u-förmig ausgebildet, so dass ein Abschnitt eines medizinischen Schlauchs 5 daran formschlüssig angebracht werden kann. In Fig. 11 und Fig. 12 sind perspektivische Ansichten von erfindungsgemäßen Stapel-Clips 19 gezeigt. Die Stapel-Clips 19 erstrecken sich jeweils in beide Richtungen von der Ebene des Aufnahmeabschnitts 7 weg und weisen jeweils zwei parallel zu dem Aufnahmeabschnitt 7 angeordnete Stapelflächen 21 auf. Die Stapelflächen 21 sind in Fig. 12 nach außen gerichtet bzw. in Fig. 11 nach innen gerichtet angeordnet.

Fig. 13 zeigt eine perspektivische Ansicht eines erfindungsgemäßen Aufnahmebehälters 22, welche geeignet ist, eine Vielzahl von Sterilverpackungen 1 aufzunehmen. Der Aufnahmebehälter 22 weist einen Boden und vier Seitenflächen auf. An einer Seitenfläche ist eine Führungsschiene 23 vorgesehen, an welcher die Träger 2 der Sterilverpackung 1 angebracht sind. Die Sterilverpackungen 1 sind somit über ihren Träger 2 an der Führungsschiene 23 des Aufnahmebehälters 22 angebracht/ befestigt/ fixiert. Wie in Fig. 13 und Fig. 14 ersichtlich ist, sind die Außenabmessungen des Aufnahmebehälters 22 derart gewählt, dass eine Breite und eine Höhe des Aufnahmebehälters 22 entsprechend den Außenabmessungen der Sterilverpackung 1 ausgewählt sind. Somit wird erreicht, dass die Sterilverpackungen 1 aneinandergereiht in dem Aufnahmebehälter 22 angeordnet sind. Dadurch wird zum einen das Verpackungsvolumen des Aufnahmebehälters gering gehalten und zum anderen ist von außen für einen Benutzer stets direkt ersichtlich, wie viele Sterilverpackungen 1 sich in dem Aufnahmebehälter 22 befinden.

### Bezugszeichenliste

- 1: Sterilverpackung
- 2: Träger
- 3: Folientasche
- 4: Verpackungsinnenraum
- 5: medizinischer Schlauch
- 6: Führungshaken
- 7: Aufnahmeabschnitt
- 8: Aufnahme
- 9: Anfangsanschlussstecker
- 10: Endanschlussstecker
- 11: Vakuumkanal
- 12: Schnittstelle
- 13: Fase
- 14: Halsabschnitt
- 15: Führungsschienenaufnahmeabschnitt
- 16: Ausnehmung
- 17: Kreisausnehmung
- 18: Konnektor-Clip
- 19: Stapel-Clip
- 20: Eck-/ Endpunkt
- 21: Stapelfläche
- 22: Aufnahmebehälter
- 23: Führungsschiene

## Patentansprüche

1. Verpackungseinrichtung (1) mit einem steril verschließbaren Aufnahmeraum (4) für zu sterilisierende oder sterilisierte medizinische Produkte (5) und einem Träger (2) zum Aufhängen und/oder Führen und/oder Hantieren der Verpackungseinrichtung (1), wobei der Träger (2) in das Innere des Aufnahmeraums (4) hineinreicht, **dadurch gekennzeichnet, dass** der Träger (2) zumindest eine, vorzugsweise passgenaue, Aufnahme (8) für einen Abschnitt (9, 10) eines zu sterilisierenden oder sterilisierten medizinischen Produkts (5) aufweist, wobei die zumindest eine Aufnahme (8) nach Art eines Konnektors (18) ausgebildet ist, welcher eingerichtet ist, die zu sterilisierenden oder sterilisierten medizinischen Produkte (5) in dem Aufnahmeraum (4) aufzunehmen, zu orientieren und zu fixieren, wobei der Konnektor (18) eine Ausnehmung zum formschlüssigen Befestigen, insbesondere Einklipsen oder Einrasten, der zu sterilisierenden oder sterilisierten medizinischen Produkte (5) aufweist.

2. Verpackungseinrichtung (1) nach Anspruch 1, **dadurch gekennzeichnet, dass** der Träger (2) einen Außenführungsabschnitt, welcher insbesondere zum Aufhängen und/oder Führen und/oder Hantieren der Verpackungseinrichtung (1) ausgebildet ist, und einen Innenaufnahmeabschnitt (7), welcher zur passgenauen Aufnahme des zu sterilisierenden oder sterilisierten medizinischen Produkts (5) ausgebildet ist, aufweist, wobei der Außenführungsabschnitt außerhalb des Aufnahmeraums (4) und der Innenaufnahmeabschnitt (7) innerhalb des Aufnahmeraums (4) angeordnet sind und der Innenaufnahmeabschnitt (7) und der Außenführungsabschnitt einstückig, vorzugsweise einmaterialig und integral, insbesondere als ein im Spritzgussverfahren hergestelltes Kunststoffteil, ausgebildet sind.

3. Verpackungseinrichtung (1) nach einem der Ansprüche 1 oder 2, **dadurch gekennzeichnet, dass** der Aufnahmeraum (4) durch eine Folienverpackung, insbesondere nach Art einer Folientasche (3) mit einer Lasche zum Verschließen der Folientasche (3), ausgebildet ist, wobei an einer Schnittstelle (12), an der der Träger (2) in das Innere der Folienverpackung übergeht, eine stoffschlüssige Verbindung, insbesondere durch Verschweißen oder Verkleben, zwischen dem Träger (2) und der Folienverpackung ausgebildet ist.

4. Verpackungseinrichtung (1) nach einem der vorhergehenden Ansprüche, **dadurch gekennzeichnet, dass** der Träger (2) eine Evakuierungseinrichtung, insbesondere nach Art eines Vakuumkanals (11), aufweist, über welche zumindest ein Teilvakuum, vorzugsweise ein Vakuum, in dem Aufnahmeraum (4) erzeugbar ist.

5. Verpackungseinrichtung (1) nach einem der Ansprüche 2 bis 4, **dadurch gekennzeichnet, dass** der Außenführungsabschnitt als ein Führungshaken (6), insbesondere nach Art eines Kleiderbügelhakens oder T-förmig mit zwei seitlichen Ausnehmungen (16), zur geeigneten Orientierung und/oder Handhabung und/oder Führung der Verpackungseinrichtung (1) ausgebildet ist.

6. Verpackungseinrichtung (1) nach einem der vorhergehenden Ansprüche, **dadurch gekennzeichnet, dass** die zumindest eine Aufnahme (8) für einen Abschnitt (9, 10) eines zu sterilisierenden oder sterilisierten medizinischen Produkts (5) eine Kennzeichnung aufweist und/oder die Aufnahme (8) in ihrer Form und/oder Gestalt an den Abschnitt (9, 10) des in ihr aufgenommenen zu sterilisierenden oder sterilisierten medizinischen Produkts (5) angepasst ist.

7. Verpackungseinrichtung (1) nach einem der vorhergehenden Ansprüche, **dadurch gekennzeichnet, dass** der Träger (2) in dem Inneren des Aufnahmeraums (4) ferner zumindest zwei, vorzugsweise drei oder vier, den Aufnahmeraum (4) aufspannende Stapelabschnitte (19) zur geeigneten Stapelung und/oder Führung der Verpackungseinrichtung (1) aufweist.

8. Verpackungseinrichtung (1) nach einem der vorhergehenden Ansprüche, **dadurch gekennzeichnet, dass** der Träger (2) in dem Inneren des Aufnahmeraums (4) u-förmig zum seitlichen Schutz der in dem Aufnahmeraum (4) aufgenommenen zu sterilisierenden oder sterilisierten medizinischen Produkte (5) ausgebildet ist.

9. Sekundärverpackung mit einer Vielzahl von Verpackungseinrichtungen (1) nach einem der vorhergehenden Ansprüche, wobei die Sekundärverpackung nach Art eines Aufnahmebehälters (22) ausgebildet ist und eine Führungsschiene (23) aufweist, an welcher die Träger (2) der Verpackungseinrichtungen (1) angebracht sind.

## Claims

1. A packaging device (1) comprising a receiving compartment (4) for medical products (5) to be sterilized or having been sterilized, the receiving compartment (4) being sealable in a sterile manner; and a carrier (2) for hanging and/or guiding and/or handling the packaging device (1), wherein the carrier (2) extends into the interior of the receiving compartment (4), **characterized in that** the carrier (2) comprises at least one, preferably accurately fitting, mount (8) for a section (9, 10) of a medical product (5) to be sterilized or having been sterilized, wherein the at least one mount (8) is formed in the way of a connector (18) which is configured to receive, orientate, and fix the medical products (5) to be sterilized or having been sterilized in the receiving compartment (4), wherein the connector (18) comprises a recess for form-fittingly securing, in particular clipping or engaging the medical products (5) to be sterilized or having been sterilized.

2. The packaging device (1) according to claim 1, **characterized in that** the carrier (2) comprises an outer guiding section which is in particular configured for hanging and/or guiding and/or handling the packaging device (1) and an inner receiving section (7) which is configured for receiving the medical product (5) to be sterilized or having been sterilized in an accurately fitting manner, wherein the outer guiding section is arranged outside the receiving compartment (4) and the inner receiving section (7) is arranged inside the receiving compartment (4), and the inner receiving section (7) and the outer guiding section are formed integrally, preferably made from one material, especially as a plastic part manufactured by injection molding.

3. The packaging device (1) according to one of claims 1 or 2, **characterized in that** the receiving compartment (4) is formed by a film package, in particular in the way of a film bag (3) having a tab for closing the film bag (3), wherein at an interface (12) where the carrier (2) passes into the interior of the film package, a bonded connection, especially by welding or gluing, is formed between the carrier (2) and the film package.

4. The packaging device (1) according to one of the preceding claims, **characterized in that** the carrier (2) includes an evacuating means in the fashion of a vacuum channel (11), by which at least a partial vacuum can be produced in the receiving compartment (4).

5. The packaging device (1) according to one of claims 2 to 4, **characterized in that** the outer guiding section is configured as a guide hook (6), in particular in the manner of a hanger hook or T-shaped including two lateral recesses (16), for appropriate orientation and/or handling and/or guiding of the packaging device (1).

6. The packaging device (1) according to one of the preceding claims, **characterized in that** the at least one mount (8) for a section (9, 10) of a medical product (5) to be sterilized or having been sterilized includes a marking and/or the mount (8) is adapted as to its shape and/or configuration to the section (9, 10) of the medical product (5) to be sterilized or having been sterilized which is accommodated in said mount.

7. The packaging device (1) according to one of the preceding claims, **characterized in that** in the interior of the receiving compartment (4) the carrier (2) further includes at least two, preferably three or four, stacking sections (19) spanning the receiving compartment (4) for appropriately stacking and/or guiding the packaging device (1).

8. The packaging device (1) according to one of the preceding claims, **characterized in that** the carrier (2) is U-shaped in the interior of the receiving compartment (4) for laterally protecting the medical products (5) to be sterilized or having been sterilized which are accommodated in the receiving compartment (4).

9. A secondary packaging with a plurality of packaging devices (1) according to one of the preceding claims, wherein the secondary packaging is configured in the way of a receptacle (22) and includes a guide rail (23) on which the carriers (2) of the packaging devices (1) are arranged.

## Revendications

1. Dispositif d'emballage (1) avec un espace de logement (4) pouvant être fermé de manière stérile pour des produits médicaux (5) à stériliser ou stérilisés et un support (2) destiné à suspendre et/ou guider et/ou manipuler le dispositif d'emballage (1), dans lequel le support (2) entre dans l'intérieur de l'espace de logement (4), **caractérisé en ce que** le support (2) présente au moins un logement (8), de préférence ajusté avec précision, pour une partie (9, 10) d'un produit médical (5) à stériliser ou stérilisé, dans lequel l'au moins un logement (8) est réalisé à la façon d'un connecteur (18), lequel est conçu pour loger, pour orienter et pour fixer les produits médicaux (5) à stériliser ou stérilisés dans l'espace de logement (4), dans lequel le connecteur (18) présente un évidement pour la fixation par complémentarité de forme, en particulier le clipsage ou l'encliquetage, des produits médicaux (5) à stériliser ou stérilisés.

2. Dispositif d'emballage (1) selon la revendication 1, **caractérisé en ce que** le support (2) présente une partie de guidage extérieur, laquelle est réalisée en particulier pour suspendre et/ou guider et/ou manipuler le dispositif d'emballage (1), et une partie de logement intérieur (7), laquelle est réalisée pour loger de manière ajustée avec précision le produit médical (5) à stériliser ou stérilisé, dans lequel la partie de guidage extérieur est agencée à l'extérieur de l'espace de logement (4) et la partie de logement intérieur (7) à l'intérieur de l'espace de logement (4) et la partie de logement intérieur (7) et la partie de guidage extérieur sont réalisées d'une seule pièce, de préférence en un seul matériau et d'un seul tenant, en particulier sous la forme d'une pièce plastique fabriquée dans le procédé de moulage par injection.

3. Dispositif d'emballage (1) selon l'une quelconque des revendications 1 ou 2, **caractérisé en ce que** l'espace de logement (4) est réalisé par un emballage en film, en particulier à la manière d'un sachet en film (3) avec une languette destinée à fermer le sachet en film (3), dans lequel une liaison de matières, en particulier par soudage ou collage, est réalisée entre le support (2) et l'emballage en film au niveau d'une interface (12), au niveau de laquelle le support (2) passe dans l'intérieur de l'emballage en film.

4. Dispositif d'emballage (1) selon l'une quelconque des revendications précédentes, **caractérisé en ce que** le support (2) présente un dispositif de mise sous vide, en particulier à la façon d'un canal à vide (11), par l'intermédiaire duquel au moins un vide partiel, de préférence un vide peut être produit dans l'espace de logement (4).

5. Dispositif d'emballage (1) selon l'une quelconque des revendications 2 à 4, **caractérisé en ce que** la partie de guidage extérieur est réalisée sous la forme d'un crochet de guidage (6), en particulier à la façon d'un crochet de cintre ou en forme de T avec deux évidements (16) latéraux, pour l'orientation et/ou la manipulation et/ou le guidage adaptés du dispositif d'emballage (1).

6. Dispositif d'emballage (1) selon l'une quelconque des revendications précédentes, **caractérisé en ce que** l'au moins un logement (8) présente une identification pour une partie (9, 10) d'un produit médical (5) à stériliser ou stérilisé et/ou le logement (8) est adapté dans sa forme et/ou configuration à la partie (9, 10) du produit médical (5) à stériliser ou stérilisé logé dans celui-ci.

7. Dispositif d'emballage (1) selon l'une quelconque des revendications précédentes, **caractérisé en ce que** le support (2) à l'intérieur de l'espace de logement (4) présente en outre au moins deux, de préférence trois ou quatre parties d'empilement (19) couvrant l'espace de logement (4) pour l'empilement et/ou le guidage adaptés du dispositif d'emballage (1).

8. Dispositif d'emballage (1) selon l'une quelconque des revendications précédentes, **caractérisé en ce que** le support (2) à l'intérieur de l'espace de logement (4) est réalisé en forme de u pour protéger latéralement les produits médicaux (5) à stériliser ou stérilisés logés dans l'espace de logement (4).

9. Emballage secondaire avec une pluralité de dispositifs d'emballage (1) selon l'une quelconque des revendications précédentes, dans lequel l'emballage secondaire est réalisé à la façon d'un contenant de logement (22) et présente un rail de guidage (23), sur lequel les supports (2) des dispositifs d'emballage (1) sont montés.
